# EUROPEAN PATENT SPECIFICATION

(11) **EP 1 371 730 B1**
(45) Date of publication and mention of the grant of the patent: **08.07.2009**
(21) Application number: 02703488.3
(22) Date of filing: 22.02.2002
(51) Int. Cl.: C12N 15/48, C12N 15/62, C07K 14/065, C07K 14/155

(54) **RECOMBINANT POXVIRUS FOR CHIMERIC PROTEINS OF THE HUMAN IMMUNODEFICIENCY VIRUS**
REKOMBINANTES POCKENVIRUS FÜR CHIMÄRE PROTEINE DES MENSCHLICHEN IMMUNSCHWÄCHEVIRUS
POXVIRUS RECOMBINE POUR PROTEINES CHIMERES DU VIRUS DE L'IMMUNODEFICIENCE HUMAINE

(30) Priority: 28.02.2001 CU 572001
(43) Date of publication of application: 17.12.2003
(73) Proprietor: CENTRO DE INGENIERIA GENETICA Y BIOTECNOLOGIA (CIGB), Ciudad de la Habana 10600 (CU)
(72) Inventor: IGLESIAS PEREZ, Enrique, Habana del Este, Ciudad de La Habana 11700 (CU); VAZQUEZ BLOMQUIST, Dania, M., Kohly, Playa, Ciudad de La Habana 10600 (CU); DUARTE CANO, Carlos, A., Playa, Ciudad de La Habana 10600 (CU)
(74) Representative: Prins, Adrianus Willem
(86) International application number: PCT/CU2002/000001
(87) International publication number: WO 2002/068654

(56) References cited:
- EP-A- 0 412 766
- WO-A-98/21354
- US-A- 5 174 993
- US-A- 5 505 941
- HANKE TOMAS ET AL: "Effective induction of simian immunodeficiency virus-specific cytotoxic T lymphocytes in macaques by using a multiepitope gene and DNA prime-modified vaccinia virus Ankara boost vaccination regimen." JOURNAL OF VIROLOGY, vol. 73, num. 9, 1999, páginas 7524-7532, XP002219831 ISSN: 0022-538X
- DUARTE CARLOS A ET AL: "Epitope mapping, V-region DNA sequence, and neutralizing Fab fragments of two monoclonal antibodies against the HIV-1 V3 loop." IMMUNOTECHNOLOGY (AMSTERDAM), vol. 2, num. 1, 1996, páginas 11-20, XP002219832 ISSN: 1380-2933
- WHITTON J LINDSAY ET AL: "A "string-of-beads" vaccine, comprising linked minigenes, confers protection from lethal dose virus challenge." JOURNAL OF VIROLOGY, vol. 67, num. 1, 1993, páginas 348-352, XP002219833 ISSN: 0022-538X
- ANN L.-L.; WHITTON L.: '"A Multivalent Minigene Vaccine, Containing B-Cell, Cytotoxic T-Lymphocyte, and Th Epitopes from Several Microbes, Induces Appropriate Responses In Vivo and Confers Protection against More than One Pathogen".' JOURNAL OF VIROLOGY vol. 71, núm. 3, 1997, páginas 2292 - 2303
- WOODBERRY T. ET AL: '"Immunogenicity of a Human Immunodeficiency Virus (HIV) Polytope Vaccine Containing Multiple HLA A2 HIV CD8+: Cytotoxic T-Cell Epitopes"' JOURNAL OF VIROLOGY vol. 73, núm. 7, 1999, páginas 5320 - 5325
- IGLESIAS E. ET AL: '"Chimeric proteins containing HIV-1 T cell epitopes: Expression in E. coli, purification and induction of antibodies in mice"' J. BIOCHEM. MOL. BIOL. BIOPHYS. vol. 5, Marzo 2001, páginas 109 - 122

## Description

### Field of the Invention

The present invention is related to the field of immunology and in particular with the development of vaccines for the prevention or treatment of Acquired Immunodeficiency Syndrome (AIDS). Chimeric genes and Fowlpox Viruses expressing them, useful for the treatment and prevention of AIDS are disclosed.

### Previous Technique

HIV is the etiological agent of AIDS (Popovic M, Sarngadharan M, Read G, and Gallo RC. Science 1984, 224:497-500). This virus infects not only CD4+ T cells (Klatzman D, Barre Sinoussi F, Nugeyre MT, Dauguet C, Vilmer E, Griscelli C, Brun-Vezinet F, Rouzioux C, Gluckman, JD, Chermannn JC and Montagnier L. Science 1984, 225:59-63) but also other cell types such as macrophages, dendritic cells, microglia and epithelial cells.

HIV can escape from the host immune response in spite of the high levels of antibodies that persist through all the infection. At the long term, HIV causes profound immunodeficiency in the host, which becomes highly susceptible to the attack of opportunistic infections.

More than 36 millions persons are living with HIV/AIDS and 94% of the 16 000 daily infections occur in developing countries. (UNAIDS. Report on the global HIV/AIDS epidemic, June 2000). Due to these alarming figures and the absence of an effective and affordable treatment for this disease, there is an urgent need for the development of an HIV vaccine.

Among several characteristics of HIV that make this task difficult the more important is perhaps the high degree of genetic variability of its antigens, especially the envelope glycoprotein (gp120) where the main domains involved in the infectious process and targeted by neutralizing antibodies are located.

Vaccine candidates based on neutralizing antibodies have been able to protect against HIV in chimpanzees (Berman PW, Gregory TJ, Lavon R, Nakamura GR, Champe MA, Porter JP, Wurm FM, Hershberg RD, Cobb GK and Eichberg JW. Nature 1990, 345: 622-625; Girard M, Kieny MP, Pinter A; Barre-Sinoussi F, Nara P, Kolbe H, Kusumi K, Chaput A, Rainhart T, Muchmore E, Ronco J, Kaczorek M, Gomard E, Gluckman JC and Fultz PN, PNAS 1991, 88: 542-546). However those experiments were performed in nearly ideal conditions where the dose, route and timing of the viral challenge were very different from natural infection. Moreover, those immunogens can't protect against divergent HIV isolates and the antibodies raised fail to neutralize primary HIV isolates.

Different vaccine candidates have been evaluated in Phase I and II clinical trials (Johnston Ml. AIDS vaccine development: status and future directions. 1999. XII Colloque des Cent Gardes. Ed. Girard M and Dodet B.161-163). Most of these are based on the envelope proteins: gp160 and gp120. Only one vaccine, based on recombinant gp120 is currently undergoing efficacy evaluation in Phase III trials in Thailand and USA. Results from previous trials suggested that only very limited protection if any can be expected from this vaccine.

Due to these serious limitations to generate a humoral response able to confer protection against different HIV isolates and subtypes, the efforts of the investigators have mostly switched in the last years toward the development of vaccine candidates capable of stimulating mainly the cellular branch of the immune system and particularly cytotoxic T cells directed against HIV antigens.

Among the experimental findings that strongly suggest the clinical relevance of anti HIV CTLs are: The administration of anti CD8 monoclonal antibody to macaques previously inoculated with Simian-Human Immunodeficiency Virus (SHIV) markedly enhanced the levels of viremia (Matano T, Shibata R, Simeón C, Connors M, Lane C, Martín M, Administration of an Anti-CD8 monoclonal antibody interferes with the clearance of chimeric Simian/Human Immunodeficiency virus during primary infections of rhesus macaques, J Virol, 1998, 72, 1: 164-169); viral variants able to escape CD8+ T cell recognition are selected in both HIV-infected individuals (Borrow, P., H. Lewicki, X. Wei, M. S. Horwitz, N. Peffer, H. Meyers, J. A. Nelson, J. E. Gairin, B. H. Hahn, M. B. A. Oldstone, and G. M. Shaw. 1997. Antiviral pressure exerted by HIV-1-specific cytotoxic T lymphocytes (CTLs) during primary infection demonstrated by rapid selection of CTL escape virus. Nature Med. 3:205-211.) and SIV-infected macaques (Allen, T. M., O. C. DH, P. Jing, J. L. Dzuris, B. R. Mothe, T. U. Vogel, E. Dunphy, M. E. Liebl, C. Emerson, N. Wilson, K. J. Kunstman, X. Wang, D. B. Allison, A. L. Hughes, R. C. Desrosiers, J. D. Altman, S. M. Wolinsky, A. Sette, and D. I. Watkins. 2000. Tat-specific cytotoxic T lymphocytes select for SIV escape variants during resolution of primary viraemia. Nature. 407:386-90); SCID mice populated with PBMC from volunteers injected with HIV-1 recombinant Vaccinia Virus (VV) were protected against challenge in the absence of neutralizing antibodies (Van Kuyk R, Torbett B, Gulizia R et al, Human CTL specific for the nef protein of HIV protect hu-PBL-SCID mice from HIV infection. AIDS Res Hum Retroviruses, 1993; 9 (suppl 1:S77); a significant proportion of exposed uninfected persons display cellular immune response specific for HIV proteins, this is true for African sex workers (Rowland-Jones SL, J Sotton, K Ariyoshi, T Dong, F Gotch, s McAdams, D Whitby, S Sabally, A Gallimore, T Corrah, M Takiguchi, T Schltz, A McMichael, H Whittle. 1995. HIV-specific cytotoxic T cells in HIV-exposed but uninfected Gambian women. Nature Medicine, 1: 59-64) and children born from seropositive mothers (Rowland-Jones SL, DF Nixon, MC Aldhous, F Gotch, K Aroyoshi, N Hallam, JS Kroll, K Froebel, A McMichael. HIV specific cytotoxic T-cell activity in an HIV-exposed but uninfected infant. Lancet, 1993, 341: 860-861). Additionally long term non progressors exhibit a strong CTL response (Cao, Y, Qin L, Zhang I, Safrit J and Ho DD, New Engl J Med, 1995, 332:201-208; Riviere Y, McChesney MB, Porrot E, et al. AIDS Res Hum Retroviruses, 11:903-990); and the HLA class I type has been associated with the rate of disease progression in HIV-1-infected individuals (Carrington, M., G. W. Nelson, M. P. Martin, T. Kissner, D. Vlahov, J. J. Goedert, R. Kaslow, S. Buchbinder, K. Hoots, and O. B. SJ. 1999. HLA and HIV-1: heterozygote advantage and B*35-Cw*04 disadvantage. Science. 283:1748-52). The CTL response precedes the neutralizing antibodies in the natural infection and has been associated with the control of viremia in acute infection (Koup RA, Safrit JT, Cao Y, et al, Temporal association of cellular immune responses with the initial control of viremia in primary human immunodeficiency syndrome, J Virol, 1994; 68: 4650-4655) and progression to AIDS correlates strongly with the impairment of CTL activity. (Harrer T, Harrer E, Kalams S, Elbeik T, Staprans S, Feinberg MB, Cao Y, Ho DD, Yilma T, Caliendo A, Jonson RP, Buchbinder S, and Walker B. HIV-specific CTL- response in healthy long-term asymptomatic HIV infection. AIDS Res Hum Retroviruses, 1996, 12, 7: 585-592). Finally vaccines that induce virus-specific CD8+ T cell responses can favorably affect the outcome of infection in SIV models of HIV infection (Barouch, D. H., S. Santra, J. E. Schmitz, M. J. Kuroda, T. M. Fu, W. Wagner, M. Bilska, A. Craiu, X. X. Zheng, G. R. Krivulka, K. Beaudry, M. A. Lifton, C. E. Nickerson, W. L. Trigona, K. Punt, D. C. Freed, L. Guan, S. Dubey, D. Casimiro, A. Simon, M. E. Davies, M. Chastain, T. B. Strom, R. S. Gelman, D. C. Montefiori, M. G. Lewis, E. A. Emini, J. W. Shiver, and N. L. Letvin. 2000. Control of viremia and prevention of clinical AIDS in rhesus monkeys by cytokine-augmented DNA vaccination. Science. 290:486-92; Gallimore, A., M. Cranage, N. Cook, N. Almond, J. Bootman, E. Rud, P. Silvera, M. Dennis, T. Corcoran, J. Stott, A. McMichael, and F. Gotch. 1995. Early suppression of SIV replication by CD8+ nef-specific cytotoxic T cells in vaccinated macaques. Nature Med. 1:1167-1173.)

All this body of experimental findings strongly suggests that therapeutic and prophylactic strategies should include the induction/preservation/restoration of this arm of the immune response as at least one of their goals.

Different methodologies have been developed to generate CTLs in animals or humans. The most effective so far has been the recombinant live vectors. This method uses harmless viruses or bacteria to transport selected genes from the pathogen into the cells of the recipient to produce there the selected antigens. This procedure of gene delivering into cells maximizes the processing of CTL epitopes and their presentation by MHC-I molecules and subsequently the efficient stimulation of CTL clones in the host.

The viruses that have been more successfully used as vectors have been the poxviruses (Poxviridae family). The best-known member of this family is Vaccinia Virus (VV), which was extensively used in humans during a smallpox eradication campaign.

Several clinical trials have been carried out with W recombinant for HIV proteins (Corey L, McElrath J, Weihold K, Matthewa T, Stablein D, Grahm B, Keefer M, Schwartz D, Gorse G. Cytotoxic T Cell and Neutralizing Antibody Responses to Human Immunodeficiency Virus Type 1 Envelope with a combination vaccine regimen. J Infectious Dis, 1998, 177:301-9; Graham BS, Matthews TJ, Belshe R, Clements ML, Dolin R, Wright PF, Gorse GJ, Schwartz DH, Keefer MC, Bolognesi DP, Corey L, Stablein D, Esterlitz JR, Hu SL, Smith GE, Fast P, Koff W, J Infectious Dis, 1993, 167: 533-7). However, VV has two main limitations for human use: (1) A small percentage of vaccinated persons showed strong adverse reactions that can be lethal in the case of immune-compromised individuals (2) persons with previous history of VV vaccination respond poorly against heterologous antigens.

A solution to these drawbacks has been the use of Avipoxvirus instead of W. These are members of the poxvirus family but their replication is restricted to avian cells and its replication cycle is abortive in human cells. Two Avipoxviruses have been used with these purposes: Canarypox Virus (CPV) and Fowlpox Virus (FPV).

Avipoxviruses recombinant for various human pathogens of tumor-associated antigens induce CTL response in animals (Limbach KJ, and E Paoletti. 1996. Non-replicating expression vectors: applications in vaccines development and gene therapy. Epidemiol. Infect. 116:241-256). The use of recombinant Avipoxvirus for vaccine development has been patented in USA (Paoletti E. y cols 1992 US5174993, Paoletti E. et al 1993, US5505941) and specifically a patent application on the use of recombinant avipoxviruses for lentiviral antigens has been presented in Europe. (Paoletti E et al, EP0956360)

A CPV recombinant for HIV-1 *gag, pol* and *env* has been evaluated in Phase I and II trials in healthy volunteers (Clements-Mann ML, K Weinhold, TJ Matthews, BS Graham, GL Gorse, MC Keefer, MJ McElrath, R-H Hsieh, J Mestecky, S Zolla-Pazner, J Mascola, D Schwartz, R Siliciano, L Corey, PF Wright, R Belshe, R Dolin, S Jackson, S Xu, P Fast, MC Walker, D Stablein, J-L Excler, J Tartaglia, A-M Duliege, F Sinangil, E Paoletti. 1998. Immune responses to Human Immunodeficiency Virus (HIV) Type 1 induced by Canarypox expressing HIV-1MN gp120, HIV-1SF2 recombinant gp120, or both vaccines in seronegative adults. J Infect Dis 177: 1230-1246; Egan MA, WA Pavlat, J Tartaglia, E Paoletti, KJ Weinhold, ML Clements, RF Siliciano. 1995. Induction of Human Immunodeficiency Virus Type 1 (HIV-1)- specific cytolytic T lymphocyte responses in seronegative adults by a nonreplicating, host-range-restricted canarypox vector (ALVAC) carrying the HIV-1MN env gene. J Infect Dis 171: 1623-1627). CTLs against at least one HIV antigen were reported in 50% of vaccinated persons in a Phase I trial, 30% in a Phase II trial and less than 10% in the last Phase I trial in Uganda. This rCPV (vCP205) was created through the insertion of HIV genes in three different non-essential regions in the genome to achieve a CTL response against more than one HIV target.

On the other hand FPV has also been used to induce a CTL response in macaques against HIV antigens in combination with DNA immunization. (Robinson HL, DC Montefiori, RP Johnson, KH Manson, ML Kalish, JD Lifson, TA Rizvi, S Lu, S-L Hu, GP Mazzara, DL Panicali, JG Herndon, R Glickmanm, MA Candido, SL Lydy, MS Wyand and HM McClure. 1999. Nature Medicine, 5: 526-534). This combination of immunogens provided some level of protection in the HIV-1/macaca nemestrina infection model (Kent SJ, A Zhao, SJ Best, JD Chandler, DB Boyle, IA Ramshaw. Enhanced T-Cell immunogenicity and protective efficacy of a human immunodeficiency virus type 1 vaccine regime consisting of a consecutive priming with DNA and boosting with recombinant fowlpox virus. 1998. J Virol, 72: 10180-10188). However this animal model presents important limitations since HIV infection in M *nemestrina* is inefficient and difficult to reproduce.

It has also been reported the generation of a CTL response through the immunization with minigenes composed of a series of exact CTL epitopes from several pathogens (Whitton, L, Sheng N, Oldstone MB, and McKee T.A "string of beads" vaccine, comprising linked minigenes, confers protection from lethal-dose virus challenge, J Virol, 1993, 67, 1:348-352; Ling-Ling An and J. Lindsay Whitton, A multivalent minigene vaccine, containing B-cell, cytotoxic T-Lymphocyte and Th epitopes from several microbes, induces appropriate responses in vivo and confers protection against more than one pathogen. J Virol, 1997, 71, 3: 2292-2302).

Modified Vaccinia Ankara (MVA) recombinant for a gag derived minigene together with the whole gag gene has been used to induce a CTL response in mice (Hanke T, RV Samuel, TJ Blanchard, VC Neumann, TM Allen, JE Boyson, SA Sharpe, N Cook, GL Smith, DI Watkins, MP Cranage, AJ McMichael. 1999. Effective induction of simian immunodeficiency virus-specific cytotoxic T lymphocytes in macaques by using a multiepitope gene and DNA prime-Modified Vaccinia Virus Ankara boost vaccination regimen. J Virol, 73, 9: 7524-7532). Those minigenes consist of a string of discrete CTL epitopes from gag.

The main limitation of the minigene approach is that the combination of individual CTL epitopes only covers a limited range of HLA antigens and therefore the CTL response elicited is by definition too much restricted.

### DESCRIPTION OF THE INVENTION

The essence of the present invention is the construction of chimeric genes composed by CTL epitope-rich regions from HIV proteins, where those regions are selected from both, internal conserved proteins and regulatory proteins expressed very early in the viral life cycle.

This solution has advantages over the described HIV minigenes because it allows the simultaneous processing of overlapping CTL epitopes presented by many HLA alleles. Another advantage of this solution in comparison to other avipoxvirus recombinant for several HIV-1 proteins is that the concentration of immunologically relevant regions from several proteins in a single gene facilitates the generation of recombinant viruses, and avoid the necessity to use several antibiotic resistance systems in the same recombinant virus. Additionally it facilitates the combination of epitopes from several HIV subtypes in a single recombinant virus. The chosen regions belong to the most conserved viral proteins and to early expressed regulatory products. Those CTL epitope-rich regions are combined with conserved T helper cell epitopes flanked by two lysines to facilitate their processing by cellular proteases. Finally a B cell epitope, recognized by a monoclonal antibody, is added to facilitate the detection of the polypeptide by immunochemical techniques.

The chimeric gene is assembled by joining together different DNA fragments, some of them generated by chemical synthesis and others amplified by Polymerase Chain Reaction (PCR) using HIV genes as templates. The DNA fragments are cloned together in an appropriate plasmid vector, sequenced and translated to a poxvirus recombination vector.

More particularly, this invention refers to the gene *cr3*, which contains Th cell epitopes from HIV-1 proteins gp120, gp41 and Vpr, the epitope on the V3 loop of gp120 recognized by Mab 2C4 (Duarte CA, Perez L, Vázquez J, Duenas M, Vilarubia OL, Navea L, Valdés R, Reyes O, Montero M, Ayala M, and Gavilondo J. Epitope mapping, V region DNA sequence, and neutralizing Fab fragments of two monoclonal antibodies against the HIV-1 V3 loop. Immunotechnology 1996, 2:11-20) and CTL epitope-rich regions in proteins RT, Gag and Nef.

Those chimeric genes are inserted in the genome of a bacterial or viral live vector (e.g., poxvirus, herpesvirus, alphavirus, poliovirus, adenovirus, BCG, Salmonella), this vector preferentially being a poxvirus, and still more specifically an avipoxvirus and even more specifically FPV. Those recombinant live vectors are used to induce a TH1 immune response and cytotoxic T cells against HIV in animals or humans.

Even more specifically this invention relates to FPV recombinant for those chimeric proteins and particularly to the recombinant FPV strains denominated FPCR3 and FPSCR3gpt, which contains the chimeric gene cr3. Once assembled as described above *cr3* is cloned in a poxvirus recombination vector, in particular a FPV recombination vector. In this particular case plasmids pEFL29 y pFP67xgpt were used as recombination vectors. pEFL29 presents homologous regions to the 6kb BamHI terminal fragment of the FPB genome, which flanks the transcriptional unit in which the heterologous gene is inserted under the control of VV 7.5K promoter, and contains also the reporter gene lacZ under the control of 4b promoter of FPV. pFP67xgpt employs open reading frames 6 and 7 from the 11.2 kb BamHI region as homologous recombination signals. Those regions flank the transcriptional unit in which the heterologous gene is placed under the synthetic poxviral E/L promoter and it also contains the *gpt* gene which confers resistance to mycophenolic acid which allows the selection of recombinant viruses.

The resultant plasmids were denominated pFPCR3 and pFPSCR3gpt respectively. Those plasmids are transfected in a primary culture of Chicken Embryo Fibroblasts (CEF) using one of the several transfection techniques available in the state of the art. In this particular case the transfection is carried out using lipofectin (Sigma, USA) in CEF previously infected with the FP29 strain of FPV but other methods such as electroporation and DEAE Dextran, among others, can be used. As a result of the homologous recombination between plasmid and the corresponding non-essential regions on the FPV genome, recombinant viruses which expressed B galactosidase can be recovered in the case of pFPCR3 or resistant to mycophenolic acid in the case of pFPSCR3gpt. The presence of the selection marker allows the identification of recombinant viral plaques and their purification by several passages on CEF. The presence of the heterologous gene on the selected viruses can be verified by PCR and the expression of the protein can be verified by western blot.

This invention relates also to the use of recombinant FPV, obtained as described, to induce a TH1 immune response with CTL activity in Balb/c mice alone or in combination with a pharmaceutically accepted formulation selected from those in the state of the art.

This invention refers also to a therapeutic or preventive combination of recombinant FPV for the described chimeric genes, and particularly to FPCR3 and FPSCR3gpt, with immunomodulators or adjuvants in particular with cytokines such as IL2, IL12, IFNγ, GMSCF, GSCF, among others, which stimulate preferentially the TH1 immune response.

Particularly it refers to combination of the FPCR3 or FPSCR3gpt viruses with daily doses of IL2 in a range between 10² and 10⁷ IU in animals or humans. The daily administration of IL2 to Balb/c mice starting the day of the administration of the FPV or later potentiates the cellular immune response against CR3.

Although it refers particularly to CR3, it is in the essence of this invention that CTL rich fragments other than those in CR3 or fragments equivalent to those in CR3 but from other HIV-1 isolates can also be used.

Similarly, although it refers particularly to FP9 strain of FPV, it is in the essence of this invention that other FPV parental strains can be used to construct the recombinant viruses, as well as another avipoxviruses such as CPV, other poxviruses such as VV or MVA or still other viruses such as herpesvirus, alphavirus, adenovirus, poliovirus or even bacteria such as BCG or Salmonella.

In another embodiment of the present invention the gene can be cloned in a proper plasmid vector for expression in mammalian cells and be injected into a mammal to induce a TH1 immune response and CTL activity in combination with a pharmaceutically acceptable carrier.

In still another embodiment of the invention it is also included a therapeutic or preventive combination of those recombinant plasmids described above with immunomodulators or adjuvants such as described or still others such as liposomes, polysaccharides, lipopeptides, lipids, proteoliposomes or combinations thereof.

In still another embodiment of this invention those genes can be cloned in other plasmids for expression of the recombinant proteins in bacteria, yeast, fungi, insect or mammalian cells, plants or in the milk of transgenic non-human animals. The proteins recovered from these systems could also be used to induce a TH1 immune response and CTL activity in animals or humans when administered in an appropriate pharmaceutically acceptable carrier.

Still another embodiment of the invention includes therapeutic or preventive combinations of CR3 protein with immunomodulators or adjuvants such as described above or still others such as liposomes, polysaccharides, lipids, proteoliposomes or other adjuvants available according to the state of the art capable to potentiate the TH1 type immune response and CTL activity in animals or humans.

### DESCRIPTION OF FIGURES

Figure 1. Plasmid pEFL-cr3, for the homologous recombination in Fowlpox using the ORF-1 from the BamH1 6Kb terminal region as insertion site. The gene cr3 is under the control of VV p7.5K promoter and the reporter gene LacZ under FPV 4b promoter.
Figure 2. Plasmid pFP67xgpt, for homologous recombination in FPV using the DNA region between ORF-6 and ORF-7 from the 11.2 kb BamHI fragment as insertion site. The gene *cr3* is placed under the control of the synthetic promoter E/L and the gene *Ecogpt* under the control of W 7.5K promoter.
Figure 3. (A) PCR and (B) Western blot of three independent cr3 recombinant FPVs: (1) FPCR3.1; (2) FPCR3.2; (3) FPCR3.3; (4) FPL29; (5) DNA molecular weight marker.
Figure 4. (A) PCR with *cr3* internal oligonucleotides (B) Western blot from three independent *cr3* recombinant FPV (1) FPSCR3GPT.1; (2) FPSCR3GPT.2; (3) FPSCR3GPT.3; (4) parental virus; (5) Molecular weight marker.
Figure 5. Stability of CR3 expression assessed by Western blot. Lanes represent three independent samples of FPV infected with FPSCR3GPT from the viral stock (1,2,3) or purified by sucrose cushion (4,5,6). Lane 7 represents CEF infected with the parental virus.
Figure 6. Results from two independent ELISPOT experiments using splenocytes from mice immunized with FPSCR3gpt and P815 cells loaded with peptide 32 or infected with VV recombinant for CR3, Gag or Nef. The results are expressed as number of IFN gamma secreting cells per 10⁶ splenocytes. The values of the corresponding negative controls (P815 alone or VV WR infected) have been subtracted.
Figure 7. IFN gamma ELISPOT experiments using splenocytes from mice immunized with FPCR3 or FPSCR3gpt and P815 stably transfected with the cr3 gene. The results are expressed as number of IFN gamma secreting cells per 10⁶ splenocytes. The values of the negative controls (parental P815) have been subtracted.

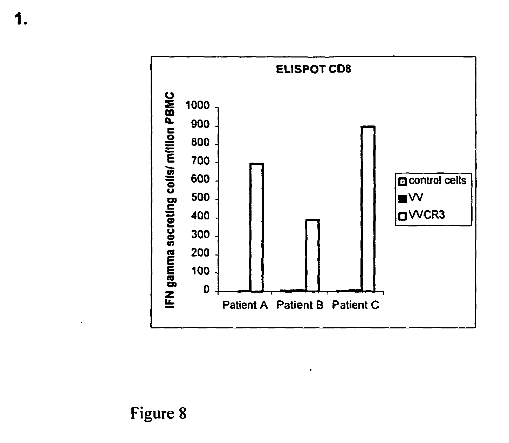

Figure 8. Recognition of VVCR3 infected autologous B cells by T lymphocytes from AIDS patients. The results from an IFNγ ELISPOT are expressed as the number of IFN gamma secreting cells per 10⁶ peripheral blood mononuclear cells.

### EXAMPLES

### Example 1. Obtention of cr3.

cr3 is a chimeric gene assembled by fragments of different HIV genes. It was assembled on pTAB11 plasmid, which is essentially equal to pTAB9 (Gómez CE, Navea L, Lobaina L, Dubed M, Expósito N, Soto A and Duarte CA. he V3 loop based Multi-Epitope Polypeptide TAB9 Adjuvated with Montanide ISA720 is Highly Immunogenic in Nonhuman Primates and Induces Neutralizing Antibodies Against Five HIV-1 isolates. Vaccine 17:2311-2319, 1999), but has the T1 and T2 T helper cell epitopes from gp120 at the 5' end extreme of the gene instead of the fragment encoding for the N-terminal part of the P64K protein. A 186 bp blunt-BamHI synthetic DNA fragment encoding for the T2 epitope from gp120, the V3 epitope of the MN strain, and T helper cell epitopes from gp41 and vpr, was cloned into pTAB11 previously digested EcoRV-BamHI. DNA sequences encoding for two consecutive lysines were inserted between individual epitopes to facilitate intracellular processing. The resultant plasmid was named pCR1. A 603 bp fragment encoding for the p66/p51 (RT) protein (pos. 2663-3109 from HIV-1 SF2 provirus) was PCR amplified using the 0.2660 and 0.2661 primers (table 1). The PCR fragment was extracted from low-melting agarose digested Bglll-EcoRl and subcloned into the Bglll-EcoRl cut pCR1 vector to obtain the pCR2 plasmid encoding for CR2 protein. Next, a 324 bp fragment, comprising a sequence of the *nef gene* (pos. 8516-8818 from HIV-1 LAI isolate), was PCR amplified with primers 0.2662 and 0.2663. Finally, another segment of 267 bp in the gag gene (pos. 1451-1696 from HIV-1 SF2) was amplified using primers 0.2664 and 0.2665 (table 1). Then, an overlapping PCR was accomplished using 20 pmol of primers 0.2662 and O. 2666 (table 1). Equal amount of each band (0.47 pmol) were mixed in PCR buffer [KCI 50 mM; Tris-HCI 10 mM, (pH 8.3), at 25°C; gelatin 0.001%], MgCl₂ 2.5 mM, dNTP 0.2 mM each and 4 U of Taq Polymerase, in a volume of 50 µL. To promote the annealing of the bands by the complementary 9 bp ends of O.2663 and O. 2664 oligonucleotides, the mixture was first heated at 92°C for 2 min and then cooled at 50°C. Finally, the temperature was increased to 72°C during 5 min to extend the annealed segments. Afterward, 10 µL of the above reaction was added to a mixture of PCR Buffer containing 2.5 mM MgCl₂, 0.2 mM dNTPs, 20 pmol of O. 2662 and 20 pmol of O.2666 and 4 U Vent pol. in 50 µL as total volume. Standard amplification conditions were 92°C for 2 min, followed by 30 cycles of 92°C for 40 sec, 50°C for 1 min and 72°C for 1 min, and a final extension at 72°C for 5 min. Next, the overlapping nef-p24 amplified band was purified from electrophoresis in low-melting agarose and digested with Xbal. Finally, the former blunt-Xbal band was cloned into a pCR2 vector previously cut Nrul-Xbal to obtain pCR3 plasmid. *cr3* encodes therefore for a chimeric proteins which includes T helper cells and CTL epitopes from gp120, gp41, vpr, RT, nef and gag presented by a wide range of HLA antigens (table 2).

**TABLE 1. DNA SEQUENCE OFOLIGONUCLEOTIDES USED IN PCR REACTIONS**

| **Oligonucleotide** | **Sequence (5'-3')** |
|---|---|
| O.2660 | GAAGATCTGTACAGAAATGGAAAAG |
| O.2661 | GGAATTCTCGCGATCCTACATACAAATCATC |
| O.2662 | GACATCACAAGTAGCAATACAGC |
| O.2663 | CCCTGCATGTGGCTCAACTGGTACTAGCTTG |
| O.2664 | GTTGAGCCACATGCAGGGCCTATTGCAC |
| O.2665 | GCTCTAGATTATTCGGCTCTTAGAGTTTTATAG |
| O.2666 | GCTCTAGATTATTCGGCTCTTAGAG |

**TABLE 2. T CELL EPITOPES IN CR₃**

| | **Epitopos** | **HLA I** | **HLAII** |
|---|---|---|---|
| **p24 87-175** | | | |
| 87-101 | HAGPIAPGQMREPRG | A2 | |
| 91-110 | IAPGQMREPRGSDIAGTTST | A2, A24, B13, B38 | |
| 101-120 | GSDIAGTTSTLQEQIGWMTN | A26, A30, B38 | |
| 108-117 | TSTLQEQIGW | B*5701,B*57,B*5801, B57, B58 | |
| 121-135 | NPPIPVGEIYKRWII | B8 | |
| 121-142 | NPPIPVGEIYKRWIILGLNKIV | B8, B27, A33, B35 | |
| 122-130 | PPIPVGEIY | B*3501 | |
| 124-138 | IPVGEIYKRWIILGL | B8 | |
| 127-135 | GEIYKRWII | B8 | |
| 128-136 | EIYKRWIIL | B8, B*0801 | |
| 129-138 | IYKRWIILGL | A*2402 | |
| 130-148 | YKRWIILGLNKTVRMYSPT | B27 | |
| 1301-139 | KRWIILGLN | B27 | |
| 134-143 | IILGLNKIVR | A33 | |
| 136-145 | LGLNKIVRMY | Bw62 | |
| 136-146 | LGLNKIVRMYS | B62 | |
| 137-145 | GLNKIVRMY | B*1501, B62 | |
| 151-170 | LDIRQGPKEPRDYVDRFYK | ND | |
| 162-172 | RDYVDRFYKTL | (B44, or A26, or B70), B*4402, A*2402 | |
| 166-174 | DRFYKTLRA | B-1402, B14 | |
| **Nef 43-150** | | | |
| 68-76 | FPVTPQVPL | B*3501, B35, B7 | |
| 68-77 | FPVTPQVPLR | B7, B*0702 | |
| 71-79 | TPQVPLRPM | B*0702 | |
| 74-81 | VPLRPMTY | B35 | |
| 73-82 | QVPLRPMTYK | A3; A11; B35 | |
| 74-81 | VPLRPMTY | B35, B*3501 | |
| 75-82 | PLRPMTYK | A*1101 | |
| 82-91 | KAAVDLSHFL | Cw8, C*0802 | |
| 83-94 | AAVDLSHFLKEK | A11 | |
| 84-91 | AVDLSHFL | Bw62 | |
| 84-92 | AVDLSHFLK | A11, A*1101 | |
| 86-94 | DLSHFLKEK | A3.1 | |
| 86-100 | DLSHFLKEKGGLEGL | A2, 835, C4 | |
| 90-97 | FLKEKGGL | B8 | |
| 92-100 | KEKGGLEGL | B60, B*4001 | |
| 93-106 | EKGGLEGLIHSQRR | A1, B8 | |
| 102-115 | HSQRRQDILDLWIY | B7 | |
| 103-127 | SQRRQDILDLWIYHTQGYFPDWQNY | B13 | |
| 105-114 | RRQDILDLWI | B*2305 | |
| 106-115 | RQDILDLWIY | B27 | |
| 115-125 | YHTQGYFPDWQ | B17 | |
| 116-125 | HTQGYFPDWQ | B57 | |
| 117-128 | TQGYFPDWQNYT | B17; B37 | |
| 117-127 | TQGYFPDWQNY | Bw62, B*1501 | |
| 120-128 | YFPDWQNYT | B*3701, B*5701, B15, B37, B57 | |
| 120-144 | YFPDWQNYTPGPGIRYPLTFGWCYK | A24 | |
| 126-137 | NYTPGPGVRYPLT | B7 | |
| 128-137 | TPGPGVRYPLT | B-0702, B*4201, B7, B7(*8101) | |
| 130-143 | GPGVRYPLTFGWCY | B*57 | |
| 132-147 | GVRYPLTFGWCYKLVP | B18, A1, B8 | |
| 133-148 | VRYPLTFGWCYKLVPV | B57 | |
| 135-143 | YPLTFGWCY | B*1801, B18, B35, B49 | |
| 136-145 | PLTFGWCYKL | A*0201, A2 | |
| **RT 36-192** | | | |
| 36-52 | EICTEMEKEGKISKIGP | ND | |
| 42-50 | EKEGKISKI | B*5101, B51 | |
| 93-101 | GIPHPAGLK | A3 | |
| 98-113 | AGLKKKKSVTVLDVGD | Cw4 | |
| 103-107 | KKSVTVLDVGDAYFS | Cw4 | |
| 107-115 | TVLDVGDAY | B35, B*3501 | |
| 108-118 | VLDVGDAYFSV | A*0201, A2 | |
| 113-120 | DAYFSVPL | B*5101, B24 | |
| 118-127 | VPLDEDFRKY | B35, B*3501 | |
| 126-135 | KYTAFTIPSI | A2 | |
| 128-135 | TAFTIPSI | B51, B*5101 | |
| 151-159 | QGWKGSPAI | B*5101 | |
| 153-165 | WKGSPAIFQSSMT | B27 | |
| 156-164 | SPAIFQSSM | B7, B35, B*3501 | |
| 158-166 | AIFQSSMTK | A*0301, A*1101, A3, A*6801, A11, A3.1, B*0301 | |
| 175-142 | KQNPDIVIY | A*3002 | |
| 177-185 | NPDIVIYQY | B35, B*3501 | |
| 181-189 | VIYQYMDDL | A2, A*0201 | |
| 181-191 | VIYQYMDDLYV | A*0201 | |
| 172-192 | FRKQNPDIVIYQYMDDLYVG | | DR1,2 6 3,4,7 |
| **T1-T2 gp120** | | | |
| 421-440 | KQIINMWQEVGKAMYAPPIE | A2 | several |
| 436-442 | KVGKAMY | A2 | |
| 436-443 | KVGKAMYA | A2 | |
| 105-117 | HEDIISLWNQSLK | A2 | several |
| 115-123 | IISLWNQSL | A2.1 | |
| **gp41** | | | |
| 584-594 | ERYLKDQQLLG | B14;B8; A24 | |
| 582-593 | YLKDQQLL | B8, B*0801, A*2402 | |
| 580-593 | ERYLKDQQLL | A*2402, B*0801, B8 | |
| 581-592 | RYLKDQQL | B14, B*1402 | |
| **Vpr** | | | |
| 66-80 | QLLFIHFRIGCRHSR | | ND |

| | | | |
|---|---|---|---|
| Numbers represent positions relative to the HXB2 amino acid sequence of each viral protein, the viral isolate is within parenthesis; ND, not defined. | | | |

### Example 2. Cloning of cr3 in pFPL29.

In pCR3, the *cr3* gene was cloned under the control of pTryp, with a Clal site on the 5'and the T4 phage gene 32 terminator and a HindIII site at 3'. This plasmid was digested Clal and Hindlll, and treated with Klenow I to obtain a *cr3* gene with ATG at the 5' end and translation stop codons at 3'. This DNA fragment was cloned in the poxvirus recombination vector pEFL29.

pEFL29 has the BamH1 6Kb terminal fragment of FPV as non-essential regions for homologous recombination in the FPV genome. This fragment contains three ORF and the ORF1 is interrupted. Flanked by these homology regions are the VV p7.5K, promoter, followed by a Smal site and the reporter gene lacZ under the control of the late promoter 4b of FPV. This plasmid includes also the kanamycin resistance gene and a bacterial origin of replication.

PEFL29 was Smal digested, treated with alkaline phosphatase and ligated with a Clall HindIII digested band containing cr3 gene. Several clones with cr3 in the right orientation under the p7.5K were selected. *E.coli* strain DH5α (φ80dlacZΔM15, recA1, endA1, gyrA96, thi-1, hsdR17 (rK- mK+), supE44, relA1, deoR, Δ(lacZYA-argF)U169) was used for propagation and selection of recombinant plasmids in LB medium containing kanamicin (25 µg/ml). All genetic manipulations were made according to Sambrook y col (Sambrook J, Fritsh EF, Maniatis T. 1989. Molecular Cloning. A Laboratory Manual. Sec Ed. Cold Spring Harbor Laboratory, Cold Spring Harbor, N.Y.)

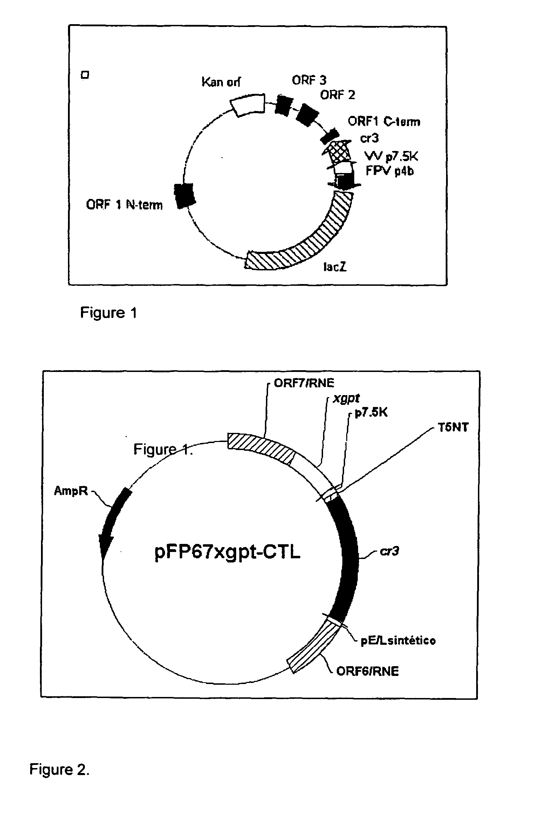

The DNA sequenced of clone pEFL-cr3 (Figure 1), was verified using an automatic sequence processor (Pharmacia). This clone was purified using CsCI gradient and used to transfect chicken embryo fibroblasts (CEF).

### Example 3. Cloning cr3 in pFP67xgpt.

*cr3* gene was PCR amplified and cloned in pMosblue vector (Amersham, UK). The resultant plasmid was named pTCR3. pTCR3 was Hpal/BamHl digested and the shorter band containing cr3 was cloned in the poxvirus vector pFP67xgpt.

pFP67xgpt has a fragment of the 11.2 Kb BamH1 of FPV genome as non-essential region for homologous recombination in the FPV genome (Tomley F, Binns M, Campwell J, Boursnell M. Sequence Analysis of an 11.2 Kilobase, near-terminal, Bam HI fragment of fowlpox virus, J Gen Virol, 1988, 69, 1025-1040). This fragment contains the open reading frame 6 and 7 of this region and the insertion occurs at the intergenic region. Flanked by these homologous regions are an E/L synthetic promoter (Carroll MW, Moss B. E. coli B-glucoronidase (GUS) as a marker for recombinant vaccinia viruses, Biotechniques, 1995, 19, 3: 352-354), and the reporter gene *Ecogpt* under the control of 7.5K promoter of VV. This plasmid includes also the kanamycin resistance gene and a bacterial origin of replication.

Plasmid pFP67xgp was cut Stul/BamHl and ligated with a cr3 containing DNA fragment derived from the Hpal/BamHl digestion of pTCR3. Several clones with *cr3* in the proper orientation under the E/L synthetic promoter were selected (Figure 1). *E.coli* strain DH5α (φ80dlacZΔM15, recA1, endA1, gyrA96, thi-1, hsdR17 (rK- mK+), supE44, relA1, deoR, Δ(lacZYA-argF)U169) was used for propagation and selection of recombinant plasmids in LB medium containing ampicillin (50 µg/ml). All genetic manipulations were made according to Sambrook et al. The DNA sequenced of clone pFP67xgptct was verified using an automatic sequence processor (Pharmacia). This clone was purified using CsCI gradient and used to transfect CEF).

### Example 4. Generation of recombinant FPVs

The parental FPV used for the generation of recombinants was the attenuated HP-438 strain, which was derived from the pathogenic strain HP-1 by six consecutive passages on CEFs, two further passages on chorioallantoic membranes, and finally 438 passages through CEFs (Mayr A and K Malicki. 1966. Attenuierung von virulentem Huhnerpockenvirus in Zellkulturen und Eigenschaften des attenuierten Virus. Zentralbl. Veterinaermed. Reihe B 13: 1-13). A twice-plaque-purified isolate of HP438 (FP9) was then passaged six times to constitute a stock. FPV stocks were grown on CEFs in 199 medium containing 2% newborn calf serum (NBCS).

Recombinant FPV were generated by homologous recombination between FP9 and plasmid pEFL29 or its derivatives as previously described. CEFs grown in 25 cm2 flasks were infected with FP9 at a multiplicity of infection (m.o.i) of 2 plaque forming units (pfu)/cell, then 2 hours later the cells were transfected with CsCl purified 10 µg of plasmid DNA (pEFL-cr3 or pFP67xgptctl) using 20 µg of Lipofectin (Gibco BRL, USA). Fresh medium (3 ml of 199 medium containing 10% tryptose phosphate broth plus 2% NBCS) was added and the cells were incubated at 37°C in a CO₂ incubator. Fresh medium was added again after 24h, and then the cells were incubated for a further 3 to 4 days. After that time, the cells were freeze-thawed three times. The cell lysate was then titrated in CEF to select the recombinant viruses. After 2hrs of adsorption the viral inoculum was removed and a layer of agarose containing EMEN was added. This layer was prepared by mixing identical volumes of 2% low melting agarose and EMEN 2X. (Gibco, Grand Island, NY) with 4% fetal calf serum (Gibco, Grand Island, NY). At day four viral plaques were evident. CEFs transfected with pEFL-cr3 were stained by adding another agarose layer with 0.33% Xgal (Melford Laboratories, UK) to the cultures. Blue plaques were selected and purified three times until 100% of viral plaques were positive for B galactosidase expression. Stocks of lacZ+ viruses were then amplified in CEF grown in 25cm2 flasks in 199 medium containing 10% tryptose phosphate broth plus 2% NBCS. The selected recombinant FPV was named FPCR3.

Selective medium for transfection with plasmid pFP67xgptctl contained mycophenolic acid (25 µg/ml), xantine (250 µg/ml) and hypoxantine (1 µg/ml). At day four viral plaques were evident. Since *gpt* and cr3 genes are flanked by the same homology regions the isolation of viral plaques in selective medium indicate that recombination occurred and both genes are inserted in FPV genome. Plaques were purified three consecutive times in CEF. The recombinant virus selected was named FPSCR3GPT.

### Example 5. PCR analysis of FPCR3.

PCR analysis was used to check that the FPCR3 recombinants contained the cr3 gene. Recombinant FPV were propagated in CEFs for 6 days and then the cells were harvested and pelleted. The pellet was suspended and incubated for 2h at 55°C in 200µl of extraction buffer (10mM Tris HCI, 100mM NaCI, 10mM EDTA, 0.5% SDS, 2% β-mercaptoethanol) containing 1.25 mg/ml of proteinase K. The DNA was then phenol-chloroform extracted and ethanol precipitated. DNA from each virus was tested by PCR with the primers described below, complementary to sequences in the 5'and 3'of cr3 gene, respectively. The PCR conditions used were 5 min at 94°C, followed by 25 cycles of 1 min at 94°C, 1 min 30 sec at 45°C and 1 min 30sec at 72°C, and a final extension at 72°C for 10 min. The primer sequences were as follows:
primer 775, 5' TATTAACATTGCCTAGTAG 3'
primer 776, 5' GAAGTAGAATCATAAAGAAC 3'

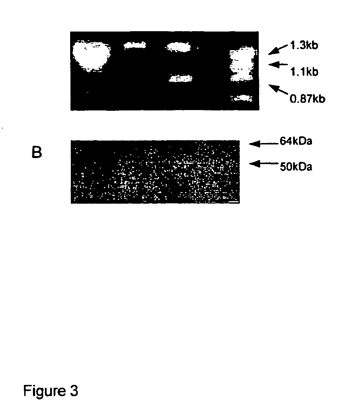

Three independent CR3 recombinant viruses (FPCR3.1; FPCR3.2; FPCR3.3), showed the expected 1.3kb band after the PCR reaction. This band was absent for the parental virus FPL29 (Figure 3A).

### Example 6. PCR analysis FPSCR3GPT

PCR analysis was used to check that the FPSCR3GPT recombinants contained the cr3 gene. Recombinant FPV were propagated in CEFs for 6 days, and then the cells were harvested and pelleted. The pellet was suspended and incubated for 2h at 55°C in 200 µl of extraction buffer (10mM Tris HCI, 100mM NaCI, 10mM EDTA, 0.5% SDS, 2% β-mercaptoethanol) containing 1.25 mg/ml of proteinase K. The DNA was then phenol-chloroform extracted and ethanol precipitated. DNA from each virus was tested by PCR with the primers described below, complementary to sequences in the 5'and 3'of cr3 gene, respectively. The PCR conditions used were 5 min at 94°C, followed by 25 cycles of 1 min at 94°C, 1 min 30 sec at 45°C and 1 min 30sec at 72°C, and a final extension at 72°C for 10 min. The primer sequences were as follows:
primer 2660, (257-279) 5' GAAGATCTGTACAGAAATGGAAAAG 3'
primer 2663, (1029-1059)5' CCCTGCATGTGGCTCAACTGGTACTAGCTTG 3'

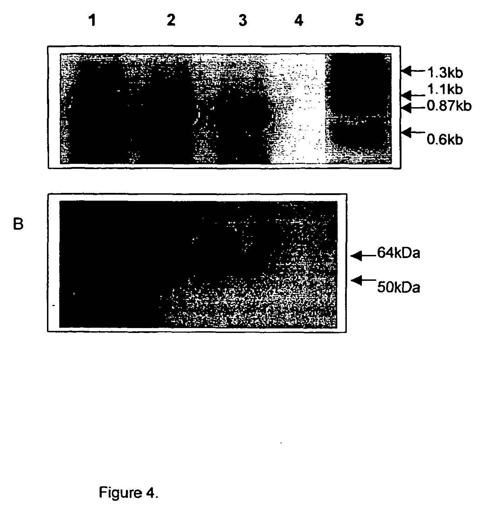

Three independent recombinant viruses (FPSCR3gpt.1; FPSCR3gpt.2; FPSCR3gpt.3), showed the expected 800 pb band after the PCR reaction. This band was absence for the parental virus FPL29 (Figure 4A).

### Example 7. Evaluation of CR3 expression by CEF infected by FPCR3.

Expression of CR3 by the FPCR3 was confirmed by Western blotting. Confluent CEFs in 60 mm Petri dishes were infected at 0.5 pfu/cell with recombinant FPV. After 24 hours the cells were harvested, pelleted and suspended in 1X SDS gel-loading buffer (50mM Tris HCI pH 6.8, 100mM DTT, 2% SDS, 0.1% bromophenol blue, 10% glycerol). Proteins were fractionated by sodium dodecyl sulphate-polyacrylamide gel electrophoresis (SDS-PAGE) on a 15% gel. They were then electro-transferred onto a nitrocellulose membrane (Hybond-C, Amersham, UK) following standard protocols. After transfer, the membrane was blocked overnight in 5% non-fat dry milk in phosphate buffered saline (PBS: 2.68mM KCI, 1.47mM KH2PO4, 0.137M NaCI, 8.06mM Na2HPO4). It was then incubated for 2h at room temperature with 10 ug/ml of monoclonal antibody 6.2, diluted in PBS containing 1% dried milk. This monoclonal antibody was produced in mice immunized with CR3 (Iglesias E, Ruiz M, Carrazana Y, Cruz LJ, Aguilar A, Jiménez V, Carpio E, Martínez M, Perez M. Martinez C, Cruz O, Martín A, Duarte C. Chimeric proteins containing HIV-1 epitopes. Journal Biochemistry, Molecular Biology and Biophysics, 2001, 5: 109-20.). The membrane was then washed and incubated with a sheep anti-mouse antibody (1:2000) conjugated to horseradish peroxidase (HRPO) (Amersham, UK). After several washes, the immunoblots were developed using the ECL Western blot detection system (Amersham, UK) according to the manufacturers' instructions. A specific band with a molecular weight between 50 y 64kDa was detected in FPCR3 infected cultures. No protein was detected in CEF infected with the parental FP9 virus (figure 3 B)

### Example 8. Evaluation of CR3 expression by CEF infected by FPSCR3qpt.

Expression of CR3 by the FPSCR3gpt was confirmed by Western blotting following a procedure similar to the one described in the previous example. A specific band with a molecular weight between 50 y 64kDa was also detected in FPSCR3gpt infected cultures while no protein was detected in CEF infected with the parental FP9 virus (figure 4 B).

### Example 9. Purification of FPCR3 and FPSCR3gpt and immunization of mice

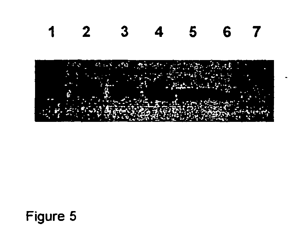

Large stocks of recombinant FPV were grown on CEFs obtained from eggs of a specific pathogen-free flock. FPV was purified by centrifugation of cytoplasmic extracts through a 25% (w/v) sucrose cushion in a Beckman SW28 rotor at 29000 rpm for 2 hours. Virus titers were then determined by plaque assay on CEF monolayers. Figure 5 shows that CR3 expression did not varies after scaling up of the culture.

Young adult (five to eight-week-old) female Balb/c mice (obtained from the SPF breeding colony at the Institute for Animal Health, Compton, UK, or the Centro Nacional de Producción de Animales de Laboratorio (CENPALAB), Cuba) were primed by the intravenous (i.v), intraperitoneal (i.p), or subcutaneous (s.c) routes with 2.5 - 5 x 10⁷ pfu of FPCR3, FPSCR3gpt or the negative control virus in 200 µl sterile PBS. Two to four weeks later, mice were boosted by the same route with a second dose of 2.5 - 9 x 10⁷ pfu of the same viruses in 200 µl sterile PBS.

### Example 10. Detection of CTL response against CR3 in Balb/c mice

Enzyme-linked-immunospot (ELISPOT) assays for detection of antigen-specific IFN-γ-releasing cells were performed using a method based on that previously described (Tanguay S and JJ Killion. Direct comparison of ELISPOT and ELISA-based assays for detection of individual cytokine-secreting cells. 1994. Lymphokine Cytokine Res, 13: 259-263). Briefly, immobilon-P membrane 96-well plates (Millipore, Molsheim, France) were coated with 100µl/well of 5µg/ml murine IFN-γ specific monoclonal antibody R4 (Pharmingen, San Diego, California) overnight at 4°C, washed 3X with PBS and blocked using RPMI 1640 medium supplemented with 10% FBS at 37°C for 1h. Test cells were then added: these were either ex vivo splenocyte suspensions (prepared as described above) from mice primed and boosted with FPCR3 or FPSCR3gpt. Different numbers of test cells were added per well: 10⁶, 2 x 10⁵ and 4 x 10⁴. Cells were stimulated by addition of P815 cells incubated with synthetic peptides at 1µM or infected with W recombinant for CR3, Gag, or Nef at a m.o.i of 5 pfu/cell. P815 cells without peptide or infected with control vaccinia viruses (vSC8 or wild type vaccinia strain WR) were included to reveal background numbers of IFN-γ-producing cells. Each well had a final volume of 200 µl of R10 medium plus hIL-2. All assay variables were tested in duplicate. After incubation overnight (at least 17 hours), the plates were washed 3X with PBS and 5X with PBS plus 0.05% Tween 20, then a secondary biotin-conjugated antibody XMG1.2 (Pharmingen, San Diego, California) was added at 0.5µg/ml and reacted at room temperature for 2h. The wells were washed 5X with PBS plus 0.05% Tween 20, and alkaline phosphatase (AP)-labeled streptavidin (Vector Labs, CA, USA) was added at a 1/1000 dilution in PBS plus 0.05% Tween 20 for 1h at room temperature. The wells were washed again 3X with PBS plus 0.05% Tween 20 and 3X with PBS, and the spots were developed using an AP activity kit (Biorad, CA, USA). After 15min, the wells were washed with tap water, dried and the spots counted under a stereoscopic microscope (Leica Microscopy System, Heerbrugg, Switzerland). Alternatively, in some assays we used HRPO-labelled streptavidin (Amersham, UK), diluted 1/800; spots were then developed with 0.1% of 3,3'-diaminobenzidine (Sigma, Saint Louis, USA) in Tris-HCI 50mM, pH 7.4 and 0.1% of hydrogen peroxide. The results were expressed as the number of spot-forming-cells (SFC) per 10⁶ splenocytes or fractionated cells. Values more than twice the negative control for each group (P815 without peptide or infected with control W) were considered positive.

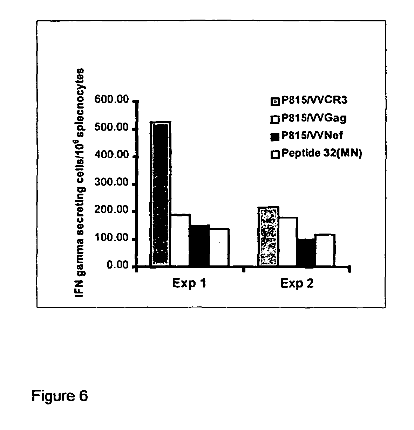

Results from two independent ELISPOT assay are shown in Figure 6. A significant fraction of splenocytes from Balb/c mice immunized with FPCR3 but no with negative virus was positive in IFN gamma ELISPOT against P815 infected either with WCR3 or Wgag and Wnef or primed with the V3 MN peptides (LKKKRIHIGPGRAFYERY).

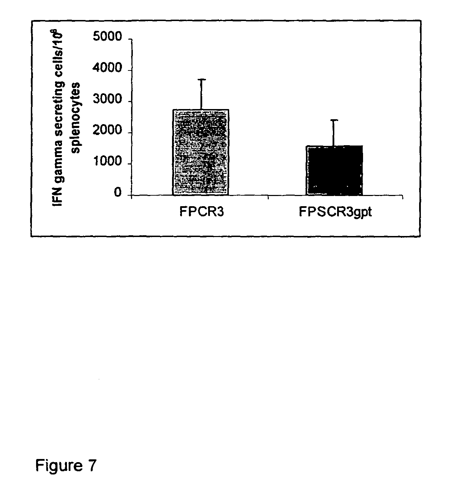

In another experiment Balb/c mice were immunized with FPCR3 or FPSCR3gpt as described and the induction of CTLs was measured using a P815 stably transfected with cr3 (P815cr3). The results from this experiment are show in figure 7. Both recombinant FPV induced a significant fraction of IFB gamma secreting cells specific for CR3.

### Example 11. Proccesing and recognition of CR3 epitopes by lymphocytes from AIDS patients.

Autologous B cells from HIV infected patients were EBV transformed and infected with a VV recombinant for CR3 (VVCR3). Those targets cells were incubated with peripheral blood lymphocytes from HIV patients and the number of IFNγ secreting splenocytes were calculated by ELISPOT. This experiment demonstrated that cr3 gene expressed by poxvirus is capable to present efficiently its epitopes to CTL lymphocytes from HIV infected patients.

## Claims

1. A chimeric gene which contains fragments from different HIV-1 genes, wherein said fragments code for overlapping cytotoxic T cell (CTL) epitopes, which are presented by a wide range of Major Histocompatiblity Complex (HLA-1) antigens, said fragments optionally further coding for T helper (Th) cell epitopes from HIV and at least one B cell epitope that is the target of a monoclonal antibody (Mab).

2. A gene according to claim 1 which codes for a chimeric polyprotein containing fragments from at least one HIV structural protein and one HIV non-structural protein.

3. A gene according to claim 2 which codes for a chimeric polyprotein containing fragments from HIV-1 proteins Reverse Transcriptase, P24 and Nef, Th epitopes from gp120, gp41 and vpr and a B cell epitope from gp120.

4. A gene according to claim 3 which codes for a chimeric polyprotein containing fragments 203-259 from Reverse Transcriptase, 219-307 from P24, and 45-147 from Nef, Th cell epitopes T1 and T2 from gp120, 580-594 from gp41 and 566-580 from vpr and a B cell epitope from the V3 region of the MN strain recognized by Mab 2C4.

5. A gene according to claim 4, which has a DNA sequence corresponding essentially with that of *cr3* gene, SEQ ID NO. 1.

6. A chimeric protein which has an amino acid sequence corresponding essentially with the sequence of the protein CR3 encoded by the the cr3 gen, SEQ ID NO 1.

7. A recombinant virus for a heterologous gene, which contains fragments from different HIV-1 genes, wherein said fragments code for overlapping CTL epitopes, which are presented by a wide range of HLA-1 antigens, said fragments optionally further coding for HIV-1 T helper cell epitopes and at least one B cell epitope recognized by a Mab.

8. A recombinant virus according to claim 7 wherein the heterologous gene codes for a chimeric protein containing fragments from at least one HIV structural protein and one HIV non-structural protein.

9. A recombinant virus according to claim 8 wherein the heterologous gene codes for a chimeric protein containing fragments from HIV-1 proteins RT, P24 and Nef, Th epitopes from gp120, gp41 and vpr and a B cell epitope from gp120.

10. A recombinant virus according to claim 9 wherein the heterologous gene codes for a chimeric protein containing fragments 203-259 from RT, 219-307 from P24, and 45-147 from NEF and Th cell epitopes T1 and T2 from gp120, 580-594 from gp41 and 566-580 from vpr and B cell epitope from the V3 region of the MN strain recognized by Mab 2C4.

11. A recombinant virus according to claim 10 wherein the DNA sequence of the heterologous gene corresponds essentially with cr3, SEQ ID NO.1.

12. A virus according to claims 7-11 wherein the virus is a poxvirus.

13. A virus according to claims 7-12 wherein the virus is an Avipoxvirus.

14. A virus according to claims 7-13 wherein the virus is Fowl Pox Virus.

15. A virus according to claims 7-14 wherein the virus is FPCR3, a Fowl Pox Virus comprising the cr3 gene.

16. A virus according to claims 7-14 wherein the virus is FPSCR3gpt, a Fowl Pox Virus comprising the cr3 gene and the gpt gene.

17. A vaccine formulation containing a recombinant virus according to claims 7 - 16 and a pharmaceutically acceptable vehicle.

18. Use of the recombinant virus according to claims 7 - 16 for manufacturing a vaccine for inducing an immune response against HIV in AIDS patients or uninfected persons.

19. A preventive or therapeutic combination composed of the vaccine formulation according to claim 17 and an immunopotentiator substance.

20. A preventive or therapeutic combination according to claim 19 wherein the immunopotentiator substance is a cytokine.

21. A preventive or therapeutic combination according to claim 20 wherein said cytokine is IL2.

22. A plasmid vector containing a chimeric gene according to claims 1-5 under the control of a mammalian cell promoter.

23. A vaccine formulation containing a recombinant plasmid vector according to claim 22 and a pharmaceutically acceptable vehicle.

24. Use of a recombinant plasmid vector according to claim 22 for manufacturing a vaccine formulation for inducing an immune response against different proteins of HIV in AIDS patients or uninfected persons.

25. A preventive or therapeutic combination composed of the vaccine formulation according to claim 23 and an immunopotentiator substance.

26. A preventive or therapeutic combination according to claim 25 wherein the immunopotentiator substance is a cytokine.

27. A preventive or therapeutic combination according to claim 26 wherein said cytokine is IL2.

## Patentansprüche

1. Chimäres Gen, welches Fragmente aus verschiedenen HIV-1-Genen enthält, wobei die besagten Fragmente für überlappende zytotoxische T-Zell (CTL)-Epitope, die von einer großen Auswahl von Haupthistokompatibilitätskomplex (HLA-1) - Antigenen präsentiert werden, kodieren, wobei die besagten Fragmente wahlweise ferner für T-Helfer-Zell (Th) -Epitope von HIV und mindestens ein B-Zell-Epitop, das Ziel eines monoklonalen Antikörpers (Mab) ist, kodieren.

2. Gen gemäß Anspruch 1, welches für ein chimäres Polyprotein, das Fragmente von mindestens einem HIV-Stukturprotein und einem HIV-Protein, bei dem es sich nicht um ein Strukturprotein handelt, enthält, kodiert.

3. Gen gemäß Anspruch 2, welches für ein chimäres Polyprotein, das Fragmente aus den HIV-1-Proteinen reverse Transkriptase, P24 und Nef, Th-Epitope aus gp120, gp41 und vpr und ein B-Zell-Epitop aus gp120 enthält, kodiert.

4. Gen gemäß Anspruch 3, welches für ein chimäres Polyprotein, das die Fragmente 203-259 aus reverser Transkriptase, 219-307 aus P24 und 45-147 aus Nef, die Th-Zell-Epitope T1 und T2 aus gp120, 580-594 aus gp41 und 566-580 aus vpr und ein B-Zell-Epitop aus der V3-Region des MN-Stammes, das von Mab 2C4 erkannt wird, enthält, kodiert.

5. Gen gemäß Anspruch 4, welches eine DNA-Sequenz, die im Wesentlichen mit der des Gens *cr3,* SEQ ID NO. 1, übereinstimmt, aufweist.

6. Chimäres Protein, welches eine Aminosäuresequenz, die im Wesentlichen mit der Sequenz des Proteins CR3, für welches das Gen *cr3,* SEQ ID NO. 1, codiert, übereinstimmt, aufweist.

7. Rekombinantes Virus für ein heterologes Gen, welches Fragmente aus verschiedenen HIV-1-Genen enthält, wobei die besagten Fragmente für überlappende CTL-Epitope, die von einer großen Auswahl von HLA-1-Antigenen präsentiert werden, kodieren, wobei die besagten Fragmente wahlweise ferner für T-Helfer-Zell-Epitope von HIV-1 und mindestens ein B-Zell-Epitop, das von einem Mab erkannt wird, kodieren.

8. Rekombinantes Virus gemäß Anspruch 7, wobei das heterologe Gen für ein chimäres Protein, das Fragmente von mindestens einem HIV-Stukturprotein und einem HIV-Protein, bei dem es sich nicht um ein Strukturprotein handelt, enthält, kodiert.

9. Rekombinantes Virus gemäß Anspruch 8, wobei das heterologe Gen für ein chimäres Protein, das Fragmente aus den HIV-1-Proteinen RT, P24 und Nef, Th-Epitope aus gp120, gp41 und vpr und ein B-Zell-Epitop aus gp120 enthält, kodiert.

10. Rekombinantes Virus gemäß Anspruch 9, wobei das heterologe Gen für ein chimäres Protein, das die Fragmente 203-259 aus RT, 219-307 aus P24 und 45-147 aus NEF und die Th-Zell-Epitope T1 und T2 aus gp120, 580-594 aus gp41 und 566-580 aus vpr und ein B-Zell-Epitop aus der V3-Region des MN-Stammes, das von Mab 2C4 erkannt wird, enthält, kodiert.

11. Rekombinantes Virus gemäß Anspruch 10, wobei die DNA-Sequenz des heterologen Gens im Wesentlichen mit *cr3,* SEQ ID NO. 1, übereinstimmt.

12. Virus gemäß den Ansprüchen 7-11, wobei es sich bei dem Virus um ein Pockenvirus handelt.

13. Virus gemäß den Ansprüchen 7-12, wobei es sich bei dem Virus um ein Vogelpockenvirus handelt.

14. Virus gemäß den Ansprüchen 7-13, wobei es sich bei dem Virus um ein Geflügelpockenvirus handelt.

15. Virus gemäß den Ansprüchen 7-14, wobei es sich bei dem Virus um FPCR3, ein Geflügelpockenvirus, welches das Gen cr3 umfasst, handelt.

16. Virus gemäß den Ansprüchen 7-14, wobei es sich bei dem Virus um FPSCR3gpt, ein Geflügelpockenvirus, welches das Gen cr3 und das Gen gpt umfasst, handelt.

17. Impfstoffformulierung, welche ein rekombinantes Virus gemäß den Ansprüchen 7-16 und einen pharmazeutisch annehmbaren Trägerstoff enthält.

18. Verwendung des rekombinanten Virus gemäß den Ansprüchen 7-16 zum Herstellen eines Impfstoffes zum Herbeiführen einer Immunantwort gegen HIV in AIDS-Patienten oder nicht infizierten Personen.

19. Vorbeugende oder therapeutische Kombination, die aus der Impfstoffformulierung gemäß Anspruch 17 und einer Immunverstärkersubstanz zusammengesetzt ist.

20. Vorbeugende oder therapeutische Kombination gemäß Anspruch 19, wobei es sich bei der Immunverstärkersubstanz um ein Zytokin handelt.

21. Vorbeugende oder therapeutische Kombination gemäß Anspruch 20, wobei es sich bei dem besagten Zytokin um IL2 handelt.

22. Plasmidvektor, welcher ein chimäres Gen gemäß den Ansprüchen 1-5 enthält, unter der Kontrolle eines Säuger-Zellpromotors.

23. Impfstoffformulierung, welche einen rekombinanten Plasmidvektor gemäß Anspruch 22 und einen pharmazeutisch annehmbaren Trägerstoff enthält.

24. Verwendung eines rekombinanten Plasmidvektors gemäß Anspruch 22 zum Herstellen einer Impfstoffformulierung zum Herbeiführen einer Immunantwort gegen verschiedene Proteine von HIV in AIDS-Patienten oder nicht infizierten Personen.

25. Vorbeugende oder therapeutische Kombination, die aus der Impfstoffformulierung gemäß Anspruch 23 und einer Immunverstärkersubstanz zusammengesetzt ist.

26. Vorbeugende oder therapeutische Kombination gemäß Anspruch 25, wobei es sich bei der Immunverstärkersubstanz um ein Zytokin handelt.

27. Vorbeugende oder therapeutische Kombination gemäß Anspruch 26, wobei es sich bei dem besagten Zytokin um IL2 handelt.

## Revendications

1. Gène chimère, qui contient des fragments de différents gènes du VIH-1, dans lequel lesdits fragments codent pour des épitopes chevauchants spécifiques aux lymphocytes T cytotoxiques (CTL), qui sont présentés par une large plage d'antigènes du complexe majeur d'histocompatibilité (HLA-1), lesdits fragments codant en outre éventuellement pour des épitopes du VIH spécifiques aux lymphocytes T auxiliaires (Th) et pour au moins un épitope spécifique aux lymphocytes B, qui est la cible d'un anticorps monoclonal (Mab).

2. Gène selon la revendication 1, qui code pour une polyprotéine chimère contenant des fragments d'au moins une protéine structurale du VIH et d'une protéine non structurale du VIH.

3. Gène selon la revendication 2, qui code pour une polyprotéine chimère contenant des fragments de protéines du VIH-1, à savoir la transcriptase inverse, la P24 et la Nef, des épitopes spécifiques aux Th de la gp120, de la gp41 et de la vpr, et un épitope spécifique aux lymphocytes B de la gp120.

4. Gène selon la revendication 3, qui code pour une polyprotéine chimère contenant le fragment 203-259 de la transcriptase inverse, le fragment 219-307 de la P24 et le fragment 45-147 de la Nef, les épitopes T1 et T2 de la gp120 spécifiques aux lymphocytes Th, le fragment 580-594 de la gp41 et le fragment 566-580 de la vpr, ainsi qu'un épitope de la région V3 de la souche MN spécifique aux lymphocytes B, reconnu par le Mab 2C4.

5. Gène selon la revendication 4, qui présente une séquence d'ADN correspondant essentiellement à celle du gène cr3 représentée par la séquence SEQ ID n°1.

6. Protéine chimère qui présente une séquence d'acides aminés correspondant essentiellement à la séquence de la protéine CR3 codée par le gène *cr*3 représenté par la séquence SEQ ID n° 1.

7. Virus recombinant pour un gène hétérologue, qui contient des fragments provenant de différents gènes du VIH-1, dans lequel lesdits fragments codent pour des épitopes chevauchants spécifiques aux CTL et qui sont présentés par une large plage d'antigènes du système HLA-1, lesdits fragments codant en outre éventuellement pour des épitopes du VIH-1 spécifiques aux lymphocytes T auxiliaires, et pour au moins un épitope spécifique aux lymphocytes B et reconnu par un Mab.

8. Virus recombinant selon la revendication 7, dans lequel le gène hétérologue code pour une protéine chimère contenant des fragments d'au moins une protéine structurale du VIH et une protéine non structurale du VIH.

9. Virus recombinant selon la revendication 8, dans lequel le gène hétérologue code pour une protéine chimère contenant des fragments des protéines RT, P24 et Nef, pour des épitopes de la gp120, de la gp41 et de la vpr spécifiques aux Th et pour un épitope de la gp120 du VIH-1 spécifique aux lymphocytes B.

10. Virus recombinant selon la revendication 9, dans lequel le gène hétérologue code pour une protéine chimère contenant le fragment 203-259 de la RT, le fragment 219-307 de la P24 et le fragment 45-147 de la NEF et les épitopes T1 et T2 de la gp120 spécifiques aux lymphocytes Th, le fragment 580-594 de la gp41 et le fragment 566-580 de la vpr, ainsi qu'un épitope de la région V3 de la souche MN et reconnu par le Mab 2C4 spécifique aux lymphocytes B.

11. Virus recombinant selon la revendication 10, dans lequel la séquence d'ADN du gène hétérologue correspond essentiellement à celle du gène *cr3* représentée par la SEQ ID n°1.

12. Virus selon les revendications 7 à 11, dans lequel le virus est un poxvirus.

13. Virus selon les revendications 7 à 12, dans lequel le virus est un poxvirus aviaire.

14. Virus selon les revendications 7 à 13, dans lequel le virus est un poxvirus du poulet.

15. Virus selon les revendications 7 à 14, dans lequel le virus est le virus FPCR3, à savoir un poxvirus du poulet comprenant le gène *cr3.*

16. Virus selon les revendications 7 à 14, dans lequel le virus est le virus FPSCR3gpt, à savoir un poxvirus du poulet comprenant le gène *cr3* et le gène *gpt.*

17. Formulation de vaccin contenant un virus recombinant selon les revendications 7 à 16 et un véhicule acceptable au plan pharmaceutique.

18. Utilisation du virus recombinant selon les revendications 7 à 16, pour la fabrication d'un vaccin destiné à induire une réponse immunitaire contre le VIH chez des patients atteints du SIDA ou chez des personnes non infectées.

19. Combinaison préventive ou thérapeutique, composée de la formulation de vaccin selon la revendication 17 et d'une substance à effet de potentialisation immunologique.

20. Combinaison préventive ou thérapeutique selon la revendication 19, dans laquelle la substance à effet de potentialisation immunologique est une cytokine.

21. Combinaison préventive ou thérapeutique selon la revendication 20, dans laquelle ladite cytokine est l'IL2.

22. Vecteur plasmidique contenant un gène chimère selon les revendications 1 à 5, placé sous le contrôle d'un promoteur provenant de cellules mammifères.

23. Formulation de vaccin contenant un vecteur plasmidique recombinant selon la revendication 22 et un véhicule acceptable au plan pharmaceutique.

24. Utilisation d'un vecteur plasmidique recombinant selon la revendication 22, pour la fabrication d'une formulation de vaccin dans le but d'induire une réponse immunitaire contre diverses protéines du VIH chez des patients atteints du SIDA ou chez des personnes non infectées.

25. Combinaison préventive ou thérapeutique, composée de la formulation de vaccin selon la revendication 23 et d'une substance à effet de potentialisation immunologique.

26. Combinaison préventive ou thérapeutique selon la revendication 25, dans laquelle la substance à effet de potentialisation immunologique est une cytokine.

27. Combinaison préventive ou thérapeutique selon la revendication 26, dans laquelle ladite cytokine est l'IL2.


### SEQUENCE LISTING

<110> CENTRO DE INGENIERIA GENETICA Y BIOTECNOLOGIA
<120> POXVIRUS RECOMBINANTES PARA PROTEINAS QUIMERICAS DEL VIRUS DE LA INMUNODEFICIECNIA HUMANA
<130> poxvirus
<150> CU 2001-0057
   <151> 2001-02-28
<160> 1
<170> PatentIn version 3.1
<210> 1
   <211> 1333
   <212> DNA
   <213> Human immunodeficiency virus
<400> 1
   atgcgtatca aacagattat caacatgtgg caggaagtgg gcaaagcgat gtatgccccg      60
   ccgatttctg gtatggttga gcagatgcat gaagatatca ttagcctgtg ggaccagtct     120
   cttaagaaaa agcgtatcca cattggccca ggccgtgcat tctatgaaag atacctaaag     180
   gatcaacagc tcctagggaa aaagcaactg ctgtttattc atttcagaat tgggtgtcga     240
   catagcagaa agaaagagat ctgtacagaa atggaaaagg aagggaaaat ttcaaaaatt     300
   gggcctgaaa atccatacaa tactccagta tttgctataa agaaaaaaga cagtactaaa     360
   tggagaaaac tagtagattt cagagaactt aataaaagaa ctcaagactt ctgggaagtt     420
   cagttaggaa taccacaccc cgcagggtta aaaaagaaaa aatcagtaac agtattggat     480
   gtgggtgatg catacttttc agttccctta gataaagact ttagaaagta tactgcattt     540
   accataccta gtataaacaa tgagacacca gggattagat atcagtacaa tgtgctgcca     600
   cagggatgga aaggatcacc agcaatattc caaagtagca tgacaaaaat cttagagcct     660
   tttagaaaac agaatccaga catagttatc tatcaataca tggatgattt gtatgtagga     720
   tcggacatca caagtagcaa tacagcagct accaatgctg attgtgcctg gctagaagca     780
   caagaggagg aggagatggg ttttccagtc acacctcagg tacctttaag accaatgact     840
   tacaaggcag ctgtagatct tagccacttt ttaaaagaaa aggggggact ggaagggcta     900
   attcactccc aacgaagaca agatatcctt gatctgtgga tctaccacac acaaggctac     960
   ttccctgatt ggcagaacta cacaccaggg ccaggggtca gatatccact gacctttgga    1020
   tggtgctaca agctagtacc agttgagcca catgcagggc ctattgcacc aggccaaatg    1080
   agagaaccaa ggggaagtga catagcagga actactagta cccttcagga acaaatagga    1140
   tggatgacaa ataatccacc tatcccagta ggagaaatct ataaaagatg gataatcctg    1200
   ggattaaata aaatagtaag aatgtatagc cctaccagct ttctggacat aagacaagga    1260
   ccaaaggaac cctttagaga ttatgtagac cggttctata aaactctaag agccgaataa    1320
   tctagaacgg atc                                                       1333